# FASCICULE DE BREVET EUROPEEN

(11) **EP 3 551 035 B1**
(45) Date de publication et mention de la délivrance du brevet: **03.09.2025**
(21) Numéro de dépôt: 17800908.0
(22) Date de dépôt: 27.10.2017
(51) Int. Cl.: A61B 3/00, A61B 3/028, A61B 3/103

(54) **PROCÉDÉ ET SYSTÈME DE MESURE DE LA RÉFRACTION, PROCÉDÉ DE CONCEPTION OPTIQUE D'UNE LENTILLE OPHTALMIQUE, ET PAIRE DE LUNETTES COMPORTANT UNE TELLE LENTILLE OPHTALMIQUE**
VERFAHREN UND SYSTEM ZUR REFRAKTIONSMESSUNG, VERFAHREN ZUM OPTISCHEN ENTWURF EINES BRILLENGLASES UND BRILLE MIT SOLCH EINEM BRILLENGLAS
METHOD AND SYSTEM FOR MEASURING REFRACTION, METHOD FOR THE OPTICAL DESIGN OF AN OPHTHALMIC LENS, AND PAIR OF GLASSES COMPRISING SUCH AN OPHTHALMIC LENS

(30) Priorité: 07.12.2016 FR 1662085
(43) Date de publication de la demande: 16.10.2019
(73) Titulaire: Essilor International, 94220 Charenton-le-Pont (FR)
(72) Inventeur: PERRIN, Jean-Luc, 94220 Charenton-Le-Pont (FR); ESCALIER, Guilhem, 94220 Charenton-Le-Pont (FR); POULAIN, Isabelle, 94220 Charenton-Le-Pont (FR); ROUSSEAU, Benjamin, 94220 Charenton-Le-Pont (FR); PETIGNAUD, Cécile, 94220 Charenton-Le-Pont (FR); MARIN, Gildas, 94220 Charenton-Le-Pont (FR)
(74) Mandataire: Jacobacci Coralis Harle
(86) Numéro de dépôt international: PCT/FR2017/052971
(87) Numéro de publication internationale: WO 2018/104602

(56) Documents cités:
- FR-A1- 2 984 716
- FR-A1- 2 992 843
- JP-A- 2016 028 760
- US-A1- 2005 105 049

## Description

La présente invention concerne de manière générale le domaine de l'optométrie et de la conception optique des lentilles ophtalmiques.

Elle concerne plus particulièrement un procédé de mesure de la réfraction d'un individu.

Elle concerne également un système adapté à la mise en œuvre de ce procédé ainsi qu'un programme d'ordinateur utilisable dans ledit système et destiné à automatiser une ou plusieurs étapes dudit procédé de mesure.

Elle concerne par ailleurs une méthode de conception optique d'une lentille ophtalmique et une méthode de sélection d'une monture de lunettes à partir de la mesure de réfraction obtenue avec ledit procédé de mesure.

Elle concerne enfin une paire de lunettes comprenant une telle lentille ophtalmique ou bien une telle monture.

### ARRIERE-PLAN TECHNOLOGIQUE

Habituellement, les mesures de la réfraction d'un individu sont réalisées avec un appareil de mesure (réfractomètre ou lunettes d'essai par exemple) dans des conditions de mesure qui s'écartent, parfois assez largement, de celles dans lesquelles l'individu portera son équipement de correction visuelle, muni d'une ou deux lentilles ophtalmiques destinées à corriger cette réfraction.

En d'autres termes, les mesures de réfraction effectuées sur l'individu sont réalisées au moyen d'un appareil de mesure standard, pour lequel les paramètres de réglage sont fixés *a priori,* sans tenir compte des éventuels paramètres visuo-posturaux de l'individu.Dans l'ensemble de cette demande, on entendra par « *paramètre visuo-postural* » un paramètre concernant la posture de l'individu en situation ou activité de vision. La posture de l'individu sera comprise au sens large comme comprenant soit une donnée relative au positionnement de sa tête (position et/ou orientation) et/ou de son ou ses yeux. Dans un sens encore plus large, la posture de l'individu pourra également être comprise comme comprenant une donnée relative au positionnement du tronc de l'individu.

Par exemple, lors d'une mesure de l'acuité visuelle en vision de près, la distance généralement utilisée pour réaliser le test visuel adapté est fixée à une valeur normalisée de 33 ou 40 centimètres. Or, il se peut que cette distance normalisée ne corresponde pas à la distance effective de lecture de l'individu lorsque celui-ci se trouve dans sa posture de lecture. Il s'ensuit donc que la mesure de réfraction est biaisée et que l'équipement de correction visuelle prescrit en fonction de cette mesure d'acuité visuelle s'avère peu ou pas adapté à restaurer une acuité suffisante.

De la même manière, la mesure de réfraction d'un individu est traditionnellement effectuée sans équipement de correction visuelle (par exemple le dernier équipement prescrit) et quasiment toujours sans la monture que l'individu pourrait choisir pour sa nouvelle prescription. Or, le port d'une monture, avec ou sans lentilles ophtalmiques, a un impact sur la posture de l'individu en conditions de porté et également sur la manière dont celui-ci effectue les tests d'acuité visuelle.

Ainsi, les mesures de réfraction usuelles ne sont pas réalisées dans des conditions de posture naturelle et non contrainte de l'individu, ni avec la monture qu'il serait susceptible de porter.

Par conséquent, les mesures de réfraction effectuées selon les procédés de mesure de l'art antérieur sont peu personnalisées et peuvent s'avérer approximatives ou, à tout le moins, peu adaptées à la prescription d'un nouvel équipement de correction visuelle.

On connaît par exemple du document FR2984716 une méthode de détermination de la réfraction d'un individu.

On connaît aussi du document FR2992843, un dispositif de mesure de réfraction oculaire et d'un paramètre géométrico-morphologique d'un individu.

### OBJET DE L'INVENTION

Afin de remédier à l'inconvénient précité de l'état de la technique, la présente invention propose un procédé de mesure de la réfraction d'un individu qui permet de tenir compte au mieux de la posture et du comportement visuel de l'individu en conditions de porté.

On propose selon l'invention un procédé de mesure de la réfraction d'un individu au moyen d'un appareil de mesure de la réfraction, conforme à la revendication 1.

Ainsi, grâce à l'utilisation d'un ou plusieurs paramètres de réglage, respectivement déduits d'un ou plusieurs paramètres visuo-posturaux de l'individu, pour le réglage préalable de l'appareil de mesure destiné à la mesure, il est possible d'effectuer cette mesure dans des conditions pour lesquelles l'individu en activité de vision est dans une posture la plus naturelle possible et adopte un comportement visuel habituel.

Grâce au réglage préalable de l'appareil de mesure avec la ou les valeurs initiales de ces paramètres de réglage, la mesure de la réfraction tient donc compte *a priori* des paramètres visuo-posturaux de l'individu et devient une mesure personnalisée de la réfraction de l'individu. Cette mesure est également plus représentative des conditions réelles de porté que l'individu pourra expérimenter avec un futur équipement de correction visuelle prescrit en correspondance avec cette mesure de réfraction personnalisée.

Ce procédé permet de converger vers une mesure personnalisée de la réfraction de l'individu qui est encore plus précise et plus représentative des conditions de porté de l'individu.

D'autres caractéristiques non limitatives et avantageuses du procédé de mesure conforme à l'invention, prises individuellement ou selon toutes les combinaisons techniquement possibles, sont énoncées dans les revendications 2 à 4.

L'invention propose également un procédé de conception optique d'une lentille ophtalmique destiné à un individu conforme à la revendication 5.

Grâce à la mise en œuvre du procédé de mesure selon l'invention, la mesure de la réfraction de l'individu est plus précise et la conception optique de la lentille ophtalmique est mieux adaptée à la correction visuelle de l'individu.

Le procédé de conception optique permet donc une meilleure personnalisation de l'équipement de correction visuelle destiné à l'individu.

De plus, ce procédé de conception optique trouve une application particulièrement avantageuse pour la conception d'une lentille ophtalmique destiné à améliorer le confort visuel d'un individu.

L'invention propose enfin un système pour la mise en œuvre du procédé de mesure de la réfraction d'un individu conforme à l'invention, conforme à la revendication 6.

Une autre invention concerne un programme d'ordinateur adapté à effectuer les calculs de l'étape b) du procédé de mesure de la réfraction d'un individu lorsqu'il est chargé et exécuté dans lesdits moyens de calcul du système précité.

### DESCRIPTION DETAILLEE D'UN EXEMPLE DE REALISATION

La description qui va suivre en regard des dessins annexés, donnés à titre d'exemples non limitatifs, fera bien comprendre en quoi consiste l'invention et comment elle peut être réalisée.

Sur les dessins annexés :
- la figure 1 représente un diagramme schématique d'un premier mode de réalisation du procédé de mesure de réfraction proposé par l'invention ;
- la figure 2 représente un diagramme schématique d'un deuxième mode de réalisation du procédé de mesure de réfraction proposé par l'invention ;
- la figure 3 représente un diagramme schématique d'un troisième mode de réalisation du procédé de mesure de réfraction proposé par l'invention ;
- la figure 4 est une vue schématique d'un individu tenant dans ses mains un dispositif de test visuel utilisable au cours de certaines étapes des procédés de mesure susmentionnés ;
- la figure 5 est une vue de face du dispositif de test de la figure 4 sur lequel est affichée une cible visuelle se déplaçant selon un protocole de suivi visuel ;
- la figure 6 est une vue schématique de la tête de l'individu et de différents plans associé à cette tête ;
- la figure 7 représente un repère lié à la tête de l'individu ;
- la figure 8 représente un afficheur du dispositif de test de la figure 4 avec une cible affichée et un repère lié à la tête de l'individu regardant la cible dans une position finale du protocole ;
- les figures 9 et 10 représentent des exemples de positions mesurées de la cible dans le référentiel lié à la tête de l'individu au cours du protocole de lecture ;
- la figure 11 est un schéma de principe montrant une direction de regard de référence dans un référentiel lié à la tête de l'individu et une surface d'affichage fictive des positions cibles théoriques ;
- la figure 12 représente dans le référentiel lié à la tête de l'individu les positions cibles théoriques sur la surface d'affichage et les positions cibles mesurées dans ce référentiel ;
- la figure 13 est un graphe illustrant le calcul de l'écart entre les positions cibles théoriques et les positions cibles mesurées ;
- la figure 14 est une courbe représentative des écarts entre les positions cibles théoriques et les positions cibles mesurées en fonction des positions cibles théoriques ;
- les figures 15 et 16 sont des courbes illustrant le calcul de paramètres du comportement visuel de l'individu lorsqu'il regarde la cible du protocole de la figure 5.

En préambule, on notera que les éléments identiques ou similaires des différents modes de réalisation représentés sur les différentes figures seront référencés par les mêmes signes de référence et ne seront pas décrits à chaque fois.

On notera également que dans l'exposé qui va suivre, les termes « haut » ou « supérieur ») et « bas » (ou « inférieur ») seront utilisés relativement à l'individu, le haut désignant le côté tourné vers la tête de l'individu et le bas désignant le côté tourné vers les pieds de l'individu.

De même, le terme « avant » désignera le côté tourné vers l'individu, le terme « arrière » désignant le côté opposé au côté avant.

Dans l'ensemble de la présente demande, on entendra par mesure de la réfraction d'un individu, la détermination des propriétés optiques de l'un et/ou l'autre des deux yeux de l'individu chez un ophtalmologue, un optométriste ou bien un opticien-lunetier.

Les propriétés optiques mesurées comprennent communément la puissance sphérique (positive ou négative), l'astigmatisme (puissance cylindrique et axe) et plus particulièrement l'astigmatisme en vision de près, l'exophorie ou l'ésophorie (puissance prismatique), le pouvoir d'accommodation (addition de puissance sphérique en vision de près). D'autres propriétés optiques peuvent encore comprendre la sensibilité aux contrastes, la sensibilité au flou, l'acuité visuelle, les aberrations optiques de l'œil d'ordre supérieur, l'acuité stéréoscopique, la vision des couleurs ou bien l'étendue du champ visuel.

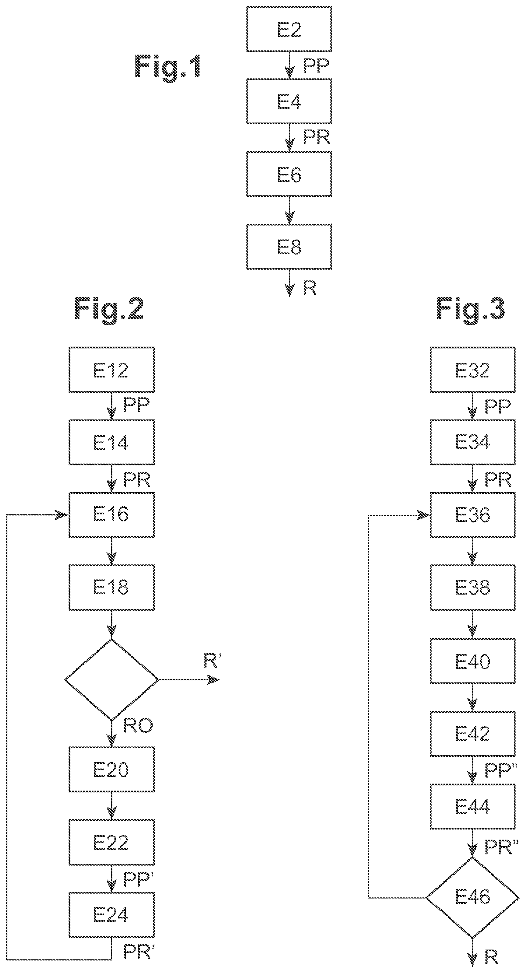

La figure 1 représente schématiquement les étapes principales d'un premier exemple de réalisation d'un procédé de mesure de réfraction conforme à l'invention.

Ce procédé débute par une étape E2 de détermination, à l'aide d'un dispositif de test visuel, d'une valeur d'un paramètre visuo-postural PP de l'individu dont on souhaite mesurer la réfraction.

Dans l'exemple décrit ici, l'individu ne porte alors aucun équipement de correction visuelle.

On pourrait toutefois prévoir en variante que l'individu porte un équipement de correction visuelle, par exemple des lunettes de correction visuelle (lunettes formées d'une monture portant au moins une lentille ophtalmique) réalisées sur la base d'une prescription antérieure, ou des lunettes d'essai (la correction apportée par ces lunettes d'essai pouvant être sélectionnée par des méthodes classiques, moins précise que la procédé de mesure de réfraction décrit ici).

Selon une autre variante, l'individu pourrait alors porter une monture nue (c'est-à-dire dépourvue de lentille ophtalmique).

Le paramètre visuo-postural PP concerné est par exemple une distance de lecture en vision de près, un angle d'abaissement du regard (en particulier en vision de près), un paramètre de convergence des deux yeux (en particulier en vision de près), ou une direction de regard (par exemple en vision de près).

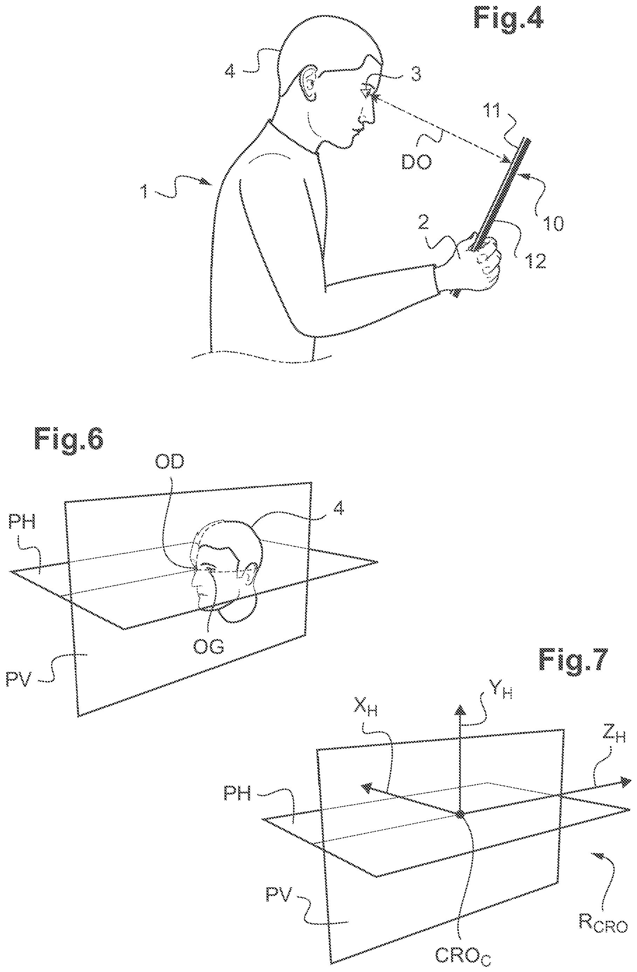

On décrit ci-après, en référence aux figures 4 à 16, un exemple de mise en œuvre possible de l'étape E2 mentionnée ci-dessus à l'aide d'un dispositif de test visuel particulier. Dans le cadre de cet exemple, le paramètre visuo-postural PP déterminé est la direction de regard (ici en vision de près) ou le barycentre de posture en vision de près ou barycentre NVB (pour « *Near-Vision Behavior* ») décrit plus loin (donnée à partir de laquelle on peut déterminer la direction du regard et/ou la distance de lecture comme indiqué plus loin).

Dans le cas où l'individu porte une monture (qu'il s'agisse d'une monture équipée ou non de lentilles ophtalmiques comme expliqué ci-dessus), on peut également mesurer à cette occasion au moins un paramètre géométrico-morphologique caractéristique du port de cette monture par l'individu (i.e. associé au couple individu - monture), tels que par exemple une distance verre-œil, un angle pantoscopique de ladite monture, un paramètre de galbe de ladite monture ou un paramètre de centrage de lentilles ophtalmiques dans ladite monture de lunettes. On peut utiliser pour ce faire un procédé tel que décrit dans le document WO 2015/101 737.

Une fois la valeur du paramètre visuo-postural PP déterminée, le procédé se poursuit à l'étape E4 en traitant cette valeur PP afin d'en déduire une valeur d'un paramètre de réglage PR associé au paramètre visuo-postural susmentionné.

Ce traitement peut être réalisé en pratique par un appareil de traitement dédié. Un tel appareil de traitement peut comprendre un module de réception de la valeur du paramètre visuo-postural PP déterminée à l'étape E2 et un module de commande d'un appareil de mesure de la réfraction, comme expliqué ci-après. Le module de réception susmentionné est par exemple conçu pour entrer en communication et échanger des données (notamment la valeur PP) avec l'appareil utilisé pour déterminer la valeur du paramètre visuo-postural PP à l'étape E2.

En variante, le traitement de la valeur du paramètre visuo-postural PP peut être réalisé par l'appareil de détermination de ce paramètre (appareil utilisé pour la mise en œuvre de l'étape E2, par exemple un appareil tel que décrit ci-après en référence aux figures 4 à 16).

Selon une autre variante encore, le traitement de la valeur du paramètre visuo-postural PP peut être réalisé par l'appareil de mesure de la réfraction utilisé pour la mise en œuvre des étapes E6 et E8 décrites ci-dessous.

Dans l'exemple décrit ici, ce traitement de l'étape E4 consiste par exemple à convertir une valeur d'abaissement du regard en vision de près en un angle d'orientation de l'appareil de mesure de la réfraction.

Dans certains modes de réalisation, le paramètre visuo-postural de l'individu est identique au paramètre de réglage de l'appareil de mesure de la réfraction ; le traitement précité peut alors consister à égaliser ladite valeur PR du paramètre de réglage à ladite valeur PP du paramètre visuo-postural.

Le traitement peut inclure en outre une transformation de la valeur mesurée PP du paramètre visuo-postural en une valeur généralement constatée correspondante du même paramètre visuo-postural, transformation effectuée selon une règle prédéfinie. On a en effet remarqué qu'il existait généralement un écart prévisible entre la valeur PP du paramètre visuo-postural mesurée lors d'un test visuel et la valeur effective de ce même paramètre visuo-postural dans des situations courantes (hors test).

Le procédé se poursuit à l'étape E6 par le réglage de l'appareil de mesure de la réfraction (déjà mentionné ci-dessus) conformément à la valeur du paramètre de réglage PR obtenue par le traitement de l'étape E4.

L'appareil de mesure de la réfraction est par exemple un réfracteur tel que décrit dans le document WO 2015/155458 ou encore dans le document WO 2015/092244. Le module de commande mentionné ci-dessus peut ainsi, après conversion d'une valeur d'abaissement du regard en un angle d'orientation, commander un actionneur du réfracteur précité afin d'orienter le support orientable du réfracteur conformément à cet angle d'orientation.

Si l'étape E4 est réalisée par un appareil de traitement dédié, la valeur du paramètre de réglage PR peut être transmise (par exemple au moyen d'un système de communication) de cet appareil de traitement à l'appareil de mesure de la réfraction. En variante, le praticien peut régler l'appareil de mesure de la réfraction en fonction d'indications relatives à la valeur du paramètre de réglage PR données par l'appareil de traitement dédié (par exemple par affichage de la valeur du paramètre de réglage PR sur un écran de l'appareil de traitement dédié).

On peut alors mettre en œuvre une étape E8 de mesure de la réfraction de l'individu au moyen de l'appareil de mesure de la réfraction, ici le réfracteur susmentionné.

La mesure de réfraction est ainsi réalisée dans des conditions de posture naturelle de l'individu de sorte que l'équipement de correction visuelle ultérieurement produit sur la base des résultats de cette mesure de réfraction sera particulièrement bien adapté à l'individu.

Dans le cas où au moins un paramètre géométrico-morphologique caractéristique du port d'une monture par l'individu a été mesuré à l'étape E2, l'appareil de mesure de la réfraction peut en outre être réglé en conformité avec l'un de ces paramètres. On peut par exemple en effet régler le réfracteur ou les lunettes d'essai de manière à ce que la position des verres d'essai corresponde au positionnement envisagé des verres (tel que défini par la monture portée durant l'étape E2).

On peut prévoir éventuellement en outre, après mise en œuvre de l'étape de mesure de réfraction précitée et lorsque l'appareil de mesure de la réfraction ne peut pas être précisément réglé sur la valeur PR du paramètre de réglage déterminée à l'étape E4, une étape finale de correction destinée à corriger la valeur de la réfraction ainsi mesurée pour tenir compte d'un écart entre la valeur PR du paramètre de réglage déterminée à l'étape E4 et la valeur à laquelle il a été possible de régler l'appareil de mesure de la réfraction lors de l'étape E6.

La mesure de réfraction de l'étape E8 permet d'obtenir une valeur de réfraction R (ou valeur de correction) associée à l'individu. En pratique, on mesure naturellement au moins une valeur de réfraction R pour chaque œil de l'individu. Comme déjà indiqué, une telle valeur de réfraction R est par exemple une valeur de puissance sphérique, une valeur de puissance cylindrique ou un axe de correction cylindrique.

La valeur de réfraction de l'individu mesurée lors de l'étape E8 est en effet ensuite utilisée, dans le cadre d'un procédé de conception optique d'une lentille ophtalmique destinée à cet individu, pour déterminer le profil optique de cette lentille ophtalmique (de sorte que ce profil optique permette d'obtenir la correction souhaitée, définie notamment par la valeur de réfraction mesurée, en tenant compte éventuellement en outre des paramètres géométrico-morphologiques susmentionnés).

La figure 2 représente schématiquement les étapes principales d'un second exemple de réalisation d'un procédé de mesure de réfraction conforme à l'invention.

Comme cela ressortira de ce qui est indiqué ci-après, les étapes E12 à E18 correspondent aux étapes E2 à E8 décrites ci-dessus en référence à la figure 1 et ne seront donc pas décrites en détail à nouveau. Les observations et variantes mentionnées dans le cadre de la description des étapes E2 à E8 peuvent s'appliquer aux étapes E12 à E18 décrites à présent.

Le procédé de la figure 2 débute par une étape initiale de détermination E12, effectuée sans équipement initial de correction visuelle, d'au moins une valeur initiale PP d'un paramètre visuo-postural de l'individu dont on souhaite mesurer la réfraction.

Le procédé comprend ensuite une étape E14 de traitement de ladite valeur initiale du paramètre visuo-postural PP pour déduire au moins une valeur initiale d'un paramètre de réglage PR associé audit paramètre visuo-postural.

Le procédé se poursuit alors par une étape E16 de réglage d'un appareil de mesure de la réfraction selon ladite valeur initiale PR du paramètre de réglage.

Le procédé comprend ensuite une étape E18 de mesure de la réfraction de l'individu au moyen dudit appareil de mesure de la réfraction tel qu'il a été réglé à l'étape E16. On obtient ainsi une valeur initiale R0 de réfraction de l'individu.

Après le premier passage à l'étape E18, le procédé se poursuit à l'étape E20, à laquelle l'individu est équipé au moyen d'un équipement de test de correction visuelle adapté à corriger la réfraction R0 mesurée à l'étape E18. (Le cas du second passage à l'étape E18 est décrit plus bas.)

Un tel équipement de test de correction visuelle est par exemple des lunettes de compensation visuelle telles que décrites dans le document WO 2015/155 456. Dans le cas où un module de commande est utilisé comme décrit ci-dessus en référence à la figure 1, le module de commande transmet par exemple des instructions à de telles lunettes de compensation visuelle afin que celles-ci génère une correction correspondant à la réfraction mesurée à l'étape E18.

Il est alors prévu, dans l'exemple décrit ici, une étape supplémentaire E22 de détermination d'au moins une valeur supplémentaire PP' du paramètre visuo-postural précité alors que l'individu est équipé de l'équipement de test. On utilise pour ce faire par exemple le dispositif de test visuel déjà mentionné pour mettre en œuvre l'étape E2, décrit ci-après en référence aux figures 4 à 16.

Le procédé peut ainsi se poursuivre par une étape supplémentaire E24 de traitement de ladite valeur supplémentaire PP' du paramètre visuo-postural pour déduire au moins une valeur suivante PR' du paramètre de réglage associé audit paramètre visuo-postural. Ce traitement est du même type que le traitement de l'étape E4 décrite ci-dessus et ne sera donc pas décrit en détail ici.

Comme visible en figure 2, le procédé boucle alors à l'étape E16, en utilisant toutefois la valeur suivante PR' du paramètre de réglage en lieu et place de la valeur initiale PR.

L'appareil de mesure de la réfraction est ainsi réglé avec la valeur suivante PR' du paramètre de réglage et peut dès lors être utilisé pour mesurer une nouvelle valeur R' de la réfraction de l'individu.

Après cette seconde mesure de réfraction, on peut mettre fin au procédé et la nouvelle valeur R' (obtenue lors du second passage à l'étape E18) peut alors être utilisée en tant que résultat du procédé de mesure.

Comme déjà indiqué, la valeur de réfraction (ici R') de l'individu obtenue grâce au procédé de mesure peut alors être utilisée, dans le cadre d'un procédé de conception optique d'une lentille ophtalmique destinée à cet individu, pour déterminer le profil optique de cette lentille ophtalmique (de sorte que ce profil optique permette d'obtenir la correction souhaitée, définie notamment par la valeur de réfraction mesurée R').

On obtient alors une lentille ophtalmique particulièrement adaptée à l'individu puisque la mesure de réfraction utilisée pour concevoir la lentille ophtalmique a non seulement été effectuée dans une posture naturelle de l'individu, mais en outre dans une situation où l'individu porte un équipement de correction visuelle, comme cela sera le cas lorsqu'il utilisera la lentille ophtalmique.

La figure 3 représente schématiquement les étapes principales d'un troisième exemple de réalisation d'un procédé de mesure de réfraction conforme à l'invention.

Comme cela ressortira de ce qui est indiqué ci-après, les étapes E32 à E38 correspondent aux étapes E2 à E8 décrites ci-dessus en référence à la figure 1 et ne seront donc pas décrites en détail à nouveau. Les observations et variantes mentionnées dans le cadre de la description des étapes E2 à E8 peuvent s'appliquer aux étapes E32 à E38 décrites à présent.

Le procédé de la figure 3 débute par une étape initiale de détermination E32, effectuée sans équipement initial de correction visuelle, d'au moins une valeur initiale PP d'un paramètre visuo-postural de l'individu dont on souhaite mesurer la réfraction.

Le procédé comprend ensuite une étape E34 de traitement de ladite valeur initiale du paramètre visuo-postural PP pour déduire au moins une valeur initiale d'un paramètre de réglage PR associé audit paramètre visuo-postural.

Le procédé se poursuit alors par une étape E36 de réglage d'un appareil de mesure de la réfraction selon ladite valeur initiale PR du paramètre de réglage.

Le procédé comprend ensuite une étape E38 de mesure de la réfraction de l'individu au moyen dudit appareil de mesure de la réfraction tel qu'il a été réglé à l'étape E36. On obtient ainsi une valeur courante R de réfraction de l'individu (qui est une valeur initiale de réfraction lors du premier passage à l'étape E38).

Le procédé se poursuit à l'étape E40, à laquelle l'individu est équipé au moyen d'un équipement de test de correction visuelle adapté à corriger la réfraction courante R mesurée lors du dernier passage à l'étape E18.

Comme dans le cas du mode de réalisation décrit ci-dessus en référence à la figure 2, un tel équipement de test de correction visuelle est par exemple des lunettes de compensation visuelle telles que décrites dans le document WO 2015/155 456. Dans le cas où un module de commande est utilisé comme décrit ci-dessus en référence à la figure 1, le module de commande transmet par exemple des instructions à de telles lunettes de compensation visuelle afin que celles-ci génère une correction correspondant à la réfraction mesurée lors du dernier passage à l'étape E38.

Il est alors procédé à une étape supplémentaire E42 de détermination d'au moins une valeur supplémentaire PP" du paramètre visuo-postural précité, l'individu étant équipé de l'équipement de test. On utilise pour ce faire par exemple le dispositif de test visuel déjà mentionné pour mettre en œuvre l'étape E2, décrit ci-après en référence aux figures 4 à 16.

Le procédé peut ainsi se poursuivre par une étape supplémentaire E44 de traitement de ladite valeur supplémentaire PP" du paramètre visuo-postural pour déduire au moins une valeur suivante PR" du paramètre de réglage associé audit paramètre visuo-postural. Ce traitement est du même type que le traitement de l'étape E4 décrite ci-dessus et ne sera donc pas décrit en détail ici.

Il est alors procédé à l'étape E46 à une étape de comparaison de la valeur initiale PR du paramètre de réglage et de la valeur suivante PR" du paramètre de réglage.

Lorsque la comparaison de l'étape E46 indique que la valeur initiale PR et la valeur suivante PR" diffèrent au-delà d'un seuil de différence prédéterminé, le procédé boucle à l'étape E36 de manière à mettre en œuvre les étapes E36 et E38 en utilisant la valeur suivante PR" du paramètre de réglage pour mesurer une nouvelle valeur de la réfraction de l'individu au moyen de l'appareil de mesure de la réfraction réglé selon cette valeur suivante PR".

On enregistre alors la nouvelle valeur de réfraction mesurée en tant que réfraction courante R et la valeur suivante PR" du paramètre de réglage en remplacement de la valeur initiale PR, puis on procède à nouveau aux étapes E40 et suivantes comme décrit ci-dessus.

Lorsque la comparaison de l'étape E46 indique au contraire que la valeur initiale PR et la valeur suivante PR" diffèrent en deçà dudit seuil de différence prédéterminé, on enregistre ladite valeur supplémentaire PP" déterminée à l'étape E42 et ladite réfraction courante R (mesurée lors du dernier passage à l'étape E38) en tant que résultats du procédé de mesure.

Cette valeur de réfraction R peut alors être utilisée, dans le cadre d'un procédé de conception optique d'une lentille ophtalmique destinée à cet individu, pour déterminer le profil optique de cette lentille ophtalmique (de sorte que ce profil optique permette d'obtenir la correction souhaitée, définie notamment par la valeur de réfraction R obtenue par le procédé qui vient d'être décrit).

Une telle lentille ophtalmique est particulièrement adaptée à l'individu puisque la mesure de réfraction utilisée pour concevoir la lentille ophtalmique a été effectuée dans une posture naturelle de l'individu dans une situation où l'individu porte un équipement de correction visuelle similaire à celui qu'il aura finalement, les conditions de mesure de réfraction s'approchant notablement des conditions de port grâce aux possibles itérations du procédé.

Comme cela ressort de la description qui précède, on utilise, pour mettre en œuvre le procédé de mesure de la réfraction d'un individu dans les modes de réalisation proposés ci-dessus, un système comprenant un dispositif de test visuel adapté à évaluer ledit paramètre visuo-postural de l'individu, des moyens de calcul adaptés pour déduire une valeur d'un paramètre de réglage à partir d'une valeur du paramètre visuo-postural de l'individu évalué par le dispositif de test visuel, et un appareil de mesure de la réfraction adapté à être réglé selon ladite valeur du paramètre de réglage déduite par les moyens de calculs et à mesurer la réfraction de l'individu.

Comme déjà indiqué, un exemple d'un tel dispositif de test est décrit plus loin en référence aux figures 4 à 16. L'appareil de mesure de la réfraction est par exemple quant à lui un réfracteur tel que décrit dans le document WO 2015/155 458.

Les moyens de calcul peuvent être intégrés à un module de commande (tel que déjà mentionné plus haut), éventuellement un module de commande dédié. En variante, comme déjà indiqué, les moyens de calcul peuvent être intégrés au dispositif de test visuel ou à l'appareil de mesure de la réfraction.

Afin d'effectuer le traitement prévu notamment à l'étape E4 ci-dessus, on peut prévoir d'utiliser un programme d'ordinateur adapté à effectuer les calculs nécessaires au traitement de l'étape E4 lorsque ce programme d'ordinateur est chargé et exécuté dans les moyens de calcul susmentionnés.

Les données obtenues au cours du procédé décrit ci-dessus (valeur du paramètre visuo-postural, réfraction mesurée) peuvent également être utilisées pour aider à la sélection d'une monture de lunettes.

Les valeurs de paramètres visuo-posturaux obtenues dans le cadre du procédé décrit ci-dessous en référence aux figures 6 à 16 peuvent être utilisées par exemple pour en déduire une taille de monture minimum afin d'aider le praticien à recommander une monture.

En particulier, il est possible grâce à la détermination d'un ou plusieurs paramètres visuo-posturaux de déduire la position finale du point de vision de près sur une lentille ophtalmique en fonction des paramètres de monture. En fonction de la longueur de progression nécessaire ou souhaitée, il est donc possible de faire une recommandation sur la taille de la monture, et plus précisément sur la taille verticale de celle-ci (taille connue sous l'appellation « côte B » dans le domaine optique).

L'individu pourra ainsi finalement porter une paire de lunettes comportant une lentille ophtalmique conçue au moyen de l'un des procédés de conception optique susmentionnés et une monture de lunettes sélectionnée comme il vient d'être décrit.

On décrit à présent en référence aux figures 4 à 16 un dispositif de test visuel utilisable pour la mise en œuvre des étapes E2, E12, E22, E32 et E42 décrites ci-dessus.

Sur la figure 4, on a représenté un individu 1 dont on souhaite tester le comportement visuel.

À cet effet, l'individu 1 tient dans ses mains 2 un dispositif de test 10 destiné à déterminer ce comportement visuel dans des conditions données, et notamment les valeurs de paramètres visuo-posturaux définissant ce comportement.

Plus particulièrement ici, on souhaite utiliser le dispositif de test 10 pour analyser de manière générale la vision de près de l'individu 1, et en particulier le comportement visuel qu'il adopte lorsqu'il est en situation de lecture.

On considérera que la vision de près correspond à une distance d'observation DO (voir figure 4) entre l'œil 3 de l'individu 1 et le dispositif de test 10 inférieure à 70 centimètres (cm).

Dans d'autres modes de réalisation, la vision intermédiaire (DO comprise entre 40 cm et 4 mètres) ou la vision de loin (DO supérieure à 4 m) peuvent être testées grâce à un tel dispositif de test.

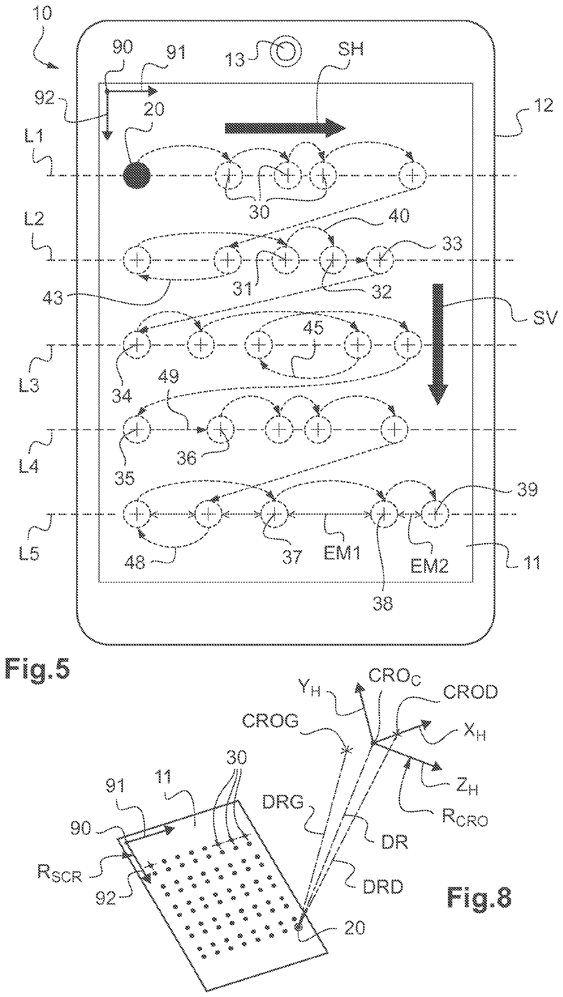

Le dispositif de test 10 comporte (voir figures 4 et 5) :
- un afficheur 11 actif qui affiche une cible 20 visuellement prépondérante en une pluralité de positions cibles 30 alignées selon au moins deux lignes ou colonnes sensiblement parallèles, et
- une unité de commande (non représentée) de l'afficheur 11, programmée pour que les positions cibles 30 définissent, au cours du temps, un protocole de suivi visuel de manière à reproduire le déplacement du regard de l'individu pendant la lecture.

L'afficheur 11 du dispositif de test peut afficher, à chaque instant du test visuel, une seule et unique cible ou bien plusieurs cibles simultanément. Dans les deux cas, la cible visuellement prépondérante est celle qui est adaptée à accrocher le regard de l'individu et que l'individu va suivre au cours du test visuel.

Lorsque plusieurs cibles sont affichées par l'afficheur 11, la cible visuellement prépondérante peut être, par exemple, une cible plus lumineuse ou plus contrastée, de couleur ou de forme (rond, carré, étoile, ...) différente, ou de taille plus petite ou plus grande que les autres, ou bien encore une cible qui clignote alors que les autres ne clignotent pas. Les différentes cibles affichées par l'afficheur peuvent également comprendre un ensemble d'indicateurs ou bien former une grille de points gris.

Dans les modes de réalisation où l'afficheur 11 n'affiche qu'une seule cible 20 (cas de la figure 5), celle-ci peut prendre une pluralité de positions cibles 30 sur l'afficheur 11. Ces positions cibles 30 sont « variables » en ce sens que la cible 20 se déplace séquentiellement d'une position cible 30 à une autre au cours du test visuel. On notera néanmoins que la séquence des positions cibles 30 prises successivement par la cible 20 dans ces modes de réalisation peut comprendre deux positions cibles 30 identiques. En d'autres termes, il est possible qu'au cours du test visuel la cible 20 repasse par une position cible 30 déjà prise précédemment.

Dans les modes de réalisation où l'afficheur affiche plusieurs cibles dont l'une est visuellement prépondérante, les positions d'affichage des cibles peuvent être variables au cours du temps, mais en tout état de cause, la cible visuellement prépondérante est celle qui se déplace selon une séquence de positions cibles de manière à imposer à l'individu 1 une succession de directions de regard particulières.

Dans la présente description, on entendra par « *protocole de suivi visuel* » la séquence d'affichage de la cible 20 visuellement prépondérante au cours du test visuel réalisé par l'individu 1.

En d'autres termes, ce protocole de suivi visuel correspond à la succession, dans le temps, des positions cibles 30 prises par la cible 20 visuellement prépondérante. Grâce à celle-ci, on impose un protocole à l'individu 1 qui regarde successivement dans une pluralité de directions particulières désirées qui sont chacune associées à une position cible 30 particulière prise par la cible 20. De cette façon, si les positions cibles 30 de cette cible 20 sont connues, il est alors possible, dans certaines conditions, de remonter à l'information concernant la direction de regard de l'individu 1 lors du test visuel.

Dans la suite de la description, on entendra par « direction de regard » de l'individu 1 associée à une position cible 30 de la cible 20, la direction de la droite passant par :
- l'un des centres de rotation de l'œil droit ou de l'œil gauche de l'individu 1, ou un barycentre de ces centres de rotation ; et
- ladite position cible 30 lorsque l'individu 1 observe la cible 20 prenant cette position cible 30.

Comme illustré sur la figure 5, le dispositif de test 10 se présente ici sous la forme d'une tablette numérique. Cette tablette numérique comprend un écran qui constitue l'afficheur 11 du dispositif de test 10. Elle comprend également un boîtier 12 entourant l'écran. L'unité de commande du dispositif de test 10 correspond, quant à elle, au contrôleur d'affichage de l'écran 11 de la tablette qui est logé à l'intérieur du boîtier 12.

Le dispositif de test 10 comporte également un appareil de capture d'image 13 qui est piloté par l'unité de commande de manière synchrone avec l'afficheur 11 pour déclencher des captures d'images de la tête 4 de l'individu 1 observant la cible 20 affichée par l'afficheur 11, chaque image capturée correspondant à une position cible 30 prédéterminée.

De préférence, on utilise ici la caméra frontale 13 intégrée à la tablette 10 comme appareil de capture d'image du dispositif de test. Cette caméra frontale 13 présente l'avantage de toujours faire face et de viser l'individu 1 lors du test visuel effectué par l'individu 1.

Dans d'autres modes de réalisation, on peut prévoir d'utiliser un appareil de capture d'image séparé et distinct de l'afficheur.

La cible 20 comprend ici un disque lumineux qui est affiché sur l'écran de la tablette, la taille de la cible étant suffisante pour qu'elle soit visible par l'individu 1 dans les conditions du test visuel. Ici, en conditions de lecture et en vision de près (DO < 70 cm), la cible 20 a une taille caractéristique (par ex. diamètre) supérieure à 5 millimètres.

De manière avantageuse, la taille caractéristique de la cible 20 est déterminée de telle sorte qu'elle puisse être vue avec une acuité supérieure à 0.1 dixième à 70 cm.

En variante, la cible peut comprendre un motif géométrique, régulier ou non. Il s'agit de préférence d'un motif quelconque, à l'exclusion d'un signe utilisé par un système d'écriture quelconque compréhensible par l'individu. En particulier, la cible visuellement prépondérante est dénuée de signification pour l'individu. Par exemple, la cible n'est pas un mot intelligible pour l'individu.

On va maintenant décrire, en référence à la figure 5, le protocole de suivi visuel qui est mis en œuvre par le dispositif de test 10 et qui est destiné ici à simuler la lecture d'un texte par l'individu 1.

De manière avantageuse, l'affichage de la cible selon le protocole de suivi visuel mis en œuvre par le dispositif de test 10 constitue un stimulus visuel pour l'individu 1, destiné à lui faire bouger les yeux 3 par suivi de cette cible 20 selon le même schéma que celui que l'individu 1 adopterait s'il lisait réellement un texte.

En d'autres termes, l'affichage de la cible 20 visuellement prépondérante sur l'afficheur 11 est commandé de telle sorte que, lorsque l'individu 1 suit du regard la cible 20 d'une position cible 30 à une autre, la direction du regard de l'individu 1 présente des directions de regard successives tout à fait similaires aux directions de regard qu'aurait cet individu 1 en lisant un texte.

La séquence des positions cibles 30 prises successivement par la cible 20 visuellement prépondérante est de préférence prédéterminée en fonction d'un texte de référence, et/ou d'un modèle de lecture, correspondant aux caractéristiques et/ou aux préférences de lecture/écriture de l'individu.

Par exemple, la séquence peut être prédéterminée préalablement avec un autre dispositif, au cours d'une opération de calibration lors de laquelle on demande à l'individu de choisir un texte de référence parmi une pluralité de textes réels disponibles et de le lire à voix haute. La vitesse de lecture peut alors servir de paramètre pour la détermination des positions d'affichage de la cible.

La séquence peut également être prédéterminée en fonction de l'âge de l'individu ou en fonction d'un niveau de lecture déclaré par l'individu, à la suite d'un questionnaire rempli par l'individu.

On peut également envisager de faire un entraînement avec une vitesse moyenne, demander à l'individu si cette vitesse moyenne était trop ou pas assez rapide et ajuster la vitesse en fonction de sa réponse.

On observera tout d'abord que la lecture d'un texte par un individu se fait naturellement selon un schéma de lecture comportant trois opérations distinctes : fixations, saccades et rétro-saccades.

Lors des fixations, l'individu déchiffre le mot qu'il est en train de lire, c'est-à-dire le mot sur lequel le regard de l'individu est fixé.

Lors des saccades, correspondant aux phases de déplacement, c'est-à-dire aux passages de la lecture d'un mot au mot suivant, les yeux de l'individu bougent rapidement pour passer d'une fixation à une autre.

Ces saccades sont liées à l'empan visuel, c'est-à-dire au nombre de caractères (lettres, symboles, idéogrammes, etc...) qui sont déchiffrables pour une fixation donnée. Elles permettent au lecteur de déchiffrer tous les caractères d'un texte.

Les saccades se font généralement dans le sens de la lecture du texte. Néanmoins, les yeux effectuent également des « rétro-saccades » très rapides dans le sens opposé au sens de lecture pour passer d'une fixation à une autre. Ce mouvement est induit par une erreur des muscles oculomoteurs ou par une mauvaise lecture et compréhension du texte.

Un des avantages du dispositif de test 10 est de proposer des protocoles de suivi visuel qui se rapprochent le plus possible des schémas de lecture de l'individu.

Le dispositif de test 10 permet donc de simuler simplement la lecture d'un texte et de placer l'individu dans une situation où il adoptera une posture naturelle proche de celle qu'il adopterait pour une lecture en vision de près.

Une détermination du comportement visuel de l'individu dans ces conditions est donc rendue plus précise et la conception optique d'une lentille ophtalmique destinée à l'individu peut être améliorée afin que le design de la lentille ophtalmique réponde aux besoins visuels de l'individu.

De préférence, les positions cibles 30 de la cible 20 sont alignées selon au moins deux lignes sensiblement parallèles. Plus précisément, sur l'exemple de réalisation montré sur les figures, l'unité de commande de l'afficheur 11 est programmée pour que les positions cibles 30 successives de la cible 20 soient alignées sur cinq lignes L1, L2, L3, L4, L5 (voir figure 5).

Alternativement, les positions cibles de la cible peuvent être alignées selon au moins deux colonnes.

De manière générale, les positions cibles 30 de la cible 20 peuvent définir des lignes parallèles de direction quelconque, notamment sensiblement horizontale ou verticale pour l'individu 1.

De préférence encore, chaque ligne, ou alternativement chaque colonne, comprend au moins trois positions alignées de ladite cible (cas des positions 37, 38, 39 pour la ligne L5 de la figure 5).

Afin que le protocole de suivi visuel soit le plus représentatif d'une lecture par le porteur, il est avantageusement prévu que le protocole de suivi visuel décrive un trajet de lecture qui soit conforme à celui défini par un système d'écriture donné, de manière à reproduire le déplacement du regard de l'individu pendant la lecture conformément au système d'écriture.

Le trajet de lecture peut être ici défini comme le chemin, au niveau de l'afficheur 11, balayé par la direction de regard de l'individu 1 lorsqu'il regarde la séquence des positions cibles 30 prises par la cible 20 visuellement prépondérante.

Le schéma de lecture adopté par un individu est lié non seulement à la nature ou aux propriétés spécifiques du texte, mais aussi aux spécificités de chaque écriture.

On notera d'ailleurs que les différentes écritures peuvent être classifiées de façon fonctionnelle (écriture alphabétique, syllabique ou logographique) et directionnelle (sens horizontal et vertical d'écriture et/ou de lecture).

On prévoit donc dans le dispositif de test que l'unité de commande mémorise un sens de parcours vertical SV et horizontal SH (voir figure 5) privilégié du protocole de suivi visuel.

Ce sens de parcours vertical et horizontal privilégié est préalablement déterminé en fonction des caractéristiques de l'individu, et en particulier sa capacité à lire un texte selon un système d'écriture donné.

Par exemple, lorsque le dispositif de test est utilisé par un français qui lit de gauche à droite et de haut en bas, le sens de parcours horizontal mémorisé par l'unité de commande est un sens de parcours allant de la gauche de l'écran 11 à la droite de l'écran 11, et le sens de parcours vertical mémorisé par l'unité de commande est un sens de parcours allant du haut de l'écran 11 au bas de l'écran 11.

Aussi, dans un mode de réalisation préféré, les lignes L1, L2, L3, L4, L5 sensiblement parallèles le long desquelles sont alignées les positions cibles 30 de la cible 20 s'étendent sensiblement horizontalement, le sens de parcours du protocole de suivi visuel étant identique pour toutes les lignes prises successivement de la plus haute à la plus basse, de gauche à droite (ou de droite à gauche pour une écriture de droite à gauche comme l'arabe ou l'hébreu).

De la même manière, lorsque le dispositif de test est utilisé par un Mongol, qui lit de haut en bas et de droite à gauche, le sens de parcours vertical mémorisé par l'unité de commande est un sens de parcours allant du haut de l'écran au bas de l'écran, et le sens de parcours horizontal mémorisé par l'unité de commande est un sens de parcours allant de la droite de l'écran à la gauche de l'écran.

Aussi, dans un mode de réalisation adapté à ce système d'écriture, les lignes sensiblement parallèles le long desquelles sont alignées les positions prédéterminées de la cible s'étendent sensiblement verticalement, le sens de parcours du protocole de suivi visuel étant identique, de haut en bas ou de bas en haut, pour toutes les lignes prises successivement de droite à gauche.

De manière avantageuse, l'unité de commande du dispositif de test 10 est programmée pour permettre la sélection du protocole de suivi visuel parmi une pluralité de protocoles de suivi visuel enregistrée dans une base de données locale ou distante, dans laquelle un sens de parcours est enregistré en association avec le protocole de suivi visuel auquel il correspond.

Ainsi, l'individu en fonction de ses caractéristiques propres de lecture et/ou d'écriture peut choisir le protocole visuel qui lui correspond, de sorte qu'il est dans des conditions naturelles proches de la lecture lors de la réalisation du test visuel. On s'assure alors de mettre en place ses mécanismes et ses stratégies de lecture afin de récupérer la posture la plus représentative de l'utilisation de sa vision de près.

Afin de reproduire le schéma de lecture tel que décrit plus haut, avec fixations, saccades et rétro-saccades, il est prévu que l'unité de commande de l'afficheur 11 affiche la cible 20 selon un protocole de suivi visuel préférentiel.

Aussi, il est prévu que l'unité de commande impose, dans chaque position cible 30 du protocole de suivi visuel, que la cible 20 s'affiche pendant une durée prédéterminée. On entend par là que la cible 20 est maintenue affichée fixement sur l'écran de manière à ce qu'on force l'individu 1 à fixer son regard sur la cible 20, ce qui correspond à une fixation sur la position cible 30 dans le trajet de lecture de l'individu 1.

De manière avantageuse, la cible 20 est fixe pendant la durée prédéterminée, c'est-à-dire que la position cible 30 de la cible 20 pendant cette durée prédéterminée ne change pas, avant le passage à la position cible suivante du trajet de lecture.

De préférence, cette durée prédéterminée est comprise entre 50 millisecondes et 1 seconde, ce qui correspond typiquement à des temps de fixation standard.

La durée prédéterminée peut également varier au cours du trajet de lecture, ceci rendant compte du fait que la fixation du regard de l'individu 1 sur un mot lors d'une lecture réelle peut dépendre du mot (taille, longueur) et du niveau de compréhension de ce mot (mot mal connu ou inconnu, mot ou caractère peu déchiffrable, mot mal orthographié, etc...).

De manière avantageuse également, il est prévu que l'unité de commande impose un délai prédéterminé entre les affichages de la cible 20 dans deux positions cibles successives (voir par exemple les positions cibles 31, 32 sur la figure 5) du protocole de suivi visuel.

De cette façon, on peut simuler grâce au dispositif de test 10 les saccades ou rétro-saccades existant lors du trajet de lecture de l'individu 1. Comme précédemment, on peut prévoir que l'unité de commande fasse varier le délai prédéterminé au cours du protocole de suivi visuel.

Ceci permet de rendre compte que la vitesse de lecture de l'individu 1 peut varier au cours de la lecture d'un texte.

Ceci permet également d'envisager les cas où la direction de regard de l'individu 1 passe d'une ligne à une autre, comme c'est le cas par exemple de la position cible 33 à la position cible 34 de la figure 5, le retour à la ligne nécessitant plus de temps dans la mesure où la variation de direction de regard de l'individu 1 est plus importante.

Il est alors possible de prévoir deux cas pour la cible lors du délai prédéterminé.

Dans un mode de réalisation, on peut prévoir que la cible est invisible pendant le délai prédéterminé. Ceci correspond au cas des positions cibles 31 et 32 de la figure 5 où la cible 20 « *saute* » (le saut étant représenté par la flèche pointillée 40) de la position 31 à la position suivante 32. Ce mode de réalisation permet de rendre compte du regard de l'individu qui saute de mot en mot lors de la lecture d'un texte.

Dans un mode de réalisation alternatif, on peut prévoir que la cible est visible pendant le délai prédéterminé et se déplace entre les deux positions cibles successives correspondantes du protocole de suivi visuel, de l'une à l'autre. Ceci correspond au cas des positions cibles 35 et 36 où la cible se déplace (le déplacement étant représenté par la flèche en pointillés 49), tout en restant visible.

De manière avantageuse, le dispositif de test 10 de l'invention est tel que l'unité de commande impose que deux positions cibles 37, 38, 39 successives du protocole de suivi visuel sont séparées d'une distance EM1, EM2 inférieure à 10 centimètres. De cette façon, lors du test visuel, on ne sollicite pas l'individu 1 de telle sorte que la variation de sa direction de regard ne soit pas trop importante, ce qui en condition de lecture est généralement le cas.

Préférentiellement, il est par ailleurs prévu que l'unité de commande impose que la distance EM1, EM2 séparant deux positions cibles 37, 38, 39 successives du protocole de suivi visuel varie le long du protocole de suivi visuel. Ceci permet d'adapter l'écart entre les cibles 20 affichées en fonction de l'empan moyen des mots pour un système d'écriture donné.

Dans un autre mode de réalisation, l'unité de commande est programmée pour que l'affichage de la cible 20 dans deux positions cibles successives du protocole de suivi visuel suive le sens de parcours privilégié, horizontal et/ou vertical, au moins six fois sur dix. Ceci est illustré sur la figure 5 sur laquelle on a représenté dans le protocole de suivi visuel, des sens de parcours, représentés par les flèches pointillées 43, 45, 48, qui vont non pas de gauche à droite comme le sens de parcours horizontal SH privilégié, mais de droite à gauche.

Il est ainsi possible grâce à cela de simuler les mouvements de rétro-saccades précédemment décrits lors de la lecture d'un texte par l'individu 1. En effet, ici quatre fois sur dix, le mouvement des yeux 3 de l'individu 1 suivant la cible 20 du regard entre deux positions cibles 30 successives se fait dans le sens opposé au sens privilégié de parcours.

Comme pour les mouvements de saccades détaillés ci-dessus, la cible 20 peut passer d'une position cible à la position cible suivante, dans un sens de parcours opposé au sens de parcours privilégié, soit en sautant d'une position à l'autre (cible invisible), soit en se déplaçant de l'une à l'autre (cible visible).

On va maintenant décrire, en référence aux figures 6 à 16, une méthode de détermination d'au moins un paramètre de comportement visuel de l'individu 1, ou paramètre visuo-postural ; cette méthode utilise le dispositif de test décrit ci-dessus qui est particulièrement adapté à la mise en œuvre de cette méthode.

La méthode de détermination comprend les étapes suivantes :
- une étape de sollicitation de l'individu pour qu'il effectue un test visuel au cours duquel il observe au moins une position cible,
- une étape de mesure d'une donnée représentative d'au moins une direction de regard de l'individu au cours dudit test visuel,
- une étape de détermination d'une direction de regard de référence, en fonction desdites données représentatives mesurées,
- une étape de positionnement, par rapport à ladite direction de regard de référence, d'au moins une position cible mesurée qui est déterminée en fonction de ladite donnée représentative de ladite direction de regard de l'individu mesurée au cours du test visuel.

Avantageusement, on réalise, après l'étape de positionnement, une étape de déduction, en fonction de ladite au moins une position cible mesurée, du ou des paramètre(s) visuo-postural(aux) recherché(s).

En pratique, la tablette 10, ou un ordinateur local ou distant, est programmée pour accomplir les étapes ci-dessus et détaillées ci-après.

De préférence, à l'étape de sollicitation de la méthode de détermination, l'individu 1 observe successivement différentes positions cibles 30.

L'individu 1 est donc sollicité pour observer l'écran 11 de la tablette 10 qui affiche la cible 20 visuellement prépondérante selon une séquence prédéterminée de positions cibles 30 du protocole de suivi visuel choisi tel que décrit plus haut en référence à la figure 5.

Selon une première variante de réalisation, la méthode de détermination comprend des étapes intermédiaires suivantes :
- on détermine lesdites directions de regard de l'individu au cours du test visuel dans un repère lié à la tête de l'individu,
- on détermine les coordonnées desdites positions cibles dans ledit repère lié à la tête de l'individu, et
- on détermine, à partir desdites coordonnées, un barycentre desdites positions cibles dans le repère lié à la tête de l'individu, et
- on définit ladite direction de regard de référence comme une droite reliant un centre de rotation d'un œil gauche ou droit de l'individu, ou un barycentre desdits centres de rotation, audit barycentre des positions cibles dans le repère lié à la tête de l'individu.

Comme référentiel lié à la tête 4 de l'individu 1, on peut par exemple choisir un référentiel dit « *référentiel de regard primaire* » ou « *repère CRO* », dans lequel la tête 4 de l'individu 1 présente une position et une orientation fixe et auquel on associe un repère, de préférence orthonormé, ayant une origine et trois axes non liés.

Les figures 6 et 7 illustrent comment est construit ce repère CRO.

En particulier, on a représenté sur la figure 6 un plan vertical PV correspondant à un plan sagittal de la tête 4 de l'individu 1 qui est le plan vertical passant par une médiatrice des deux yeux droit et gauche OD, OG de l'individu 1.

Cette médiatrice des yeux OD, OG est un axe qui passe au milieu d'un segment qui est défini par le centre de rotation de l'œil droit OD (ci-après référencé CROD) et le centre de rotation de l'œil gauche OG (ci-après référencé CROG) et qui est parallèle au plan de Francfort de la tête 4 de l'individu 1.

Le plan de Francfort de la tête de l'individu est défini comme le plan passant par les points orbitaires inférieurs de l'individu 1 et le porion de l'individu 1, le porion étant le point du crâne le plus élevé du conduit auditif, qui correspond au tragion de l'oreille. Pour la détermination du plan de Francfort, on considère que l'individu est dans une position orthostatique, dans laquelle il réalise un minimum d'efforts. Cette position correspond à une posture naturelle, ci-après désignée « *posture de regard primaire* ».

Dans cette position naturelle, la direction de regard de l'individu est alors la direction de regard primaire, c'est-à-dire qu'il regarde droit devant lui. Le plan de Francfort est alors généralement horizontal.

On définit par ailleurs (voir figure 6) un plan PH qui contient les centres de rotation CROD, CROG des yeux OD, OG de l'individu 1.

Dans l'exemple particulier décrit ici, ce plan PH est parallèle au plan de Francfort de la tête 4 de l'individu 1 et, est donc horizontal.

À partir de la posture de regard primaire de l'individu 1, c'est-à-dire de la connaissance de l'orientation du plan de Francfort, et des centres de rotation CROD, CROG des yeux OD, OG de l'individu 1, il est possible de construire le repère CRO lié à la tête 4 de l'individu 1, ci-après référencé R_{CRO}, en choisissant :
- une origine qui est l'un des centres de rotation CROD, CROG de l'œil droit OD ou de l'œil gauche OG de l'individu 1 ou un barycentre de ces centres de rotation CROD, CROG ;
- un premier axe qui est parallèle à une direction de regard primaire de l'individu 1 ;
- un deuxième axe qui est horizontal et perpendiculaire au premier axe, et
- un troisième axe qui est perpendiculaire au premier axe et au deuxième axe.

Dans les exemples de réalisation décrits, l'origine du repère R_{CRO} est choisie comme étant le point situé au milieu du segment joignant le centre de rotation CROD de l'œil droit OD et le centre de rotation CROG de l'œil gauche OG de l'individu 1. En d'autres termes, ce point d'origine, désigné ci-après « *CRO cyclope* » et référencé CRO_{C} correspond à l'isobarycentre des centres de rotation CROD, CROG des yeux OD, OG de l'individu 1.

Les trois axes X_{H}, Y_{H}, Z_{H}, du repère R_{CRO} sont également représentés sur la figure 7.

L'axe X_{H} (deuxième axe) passe par le CRO cyclope CRO_{C} et est ici orienté du centre de rotation gauche CROG vers le centre de rotation droit CROD. L'axe X_{H} est ici horizontal car contenu dans le plan horizontal PH parallèle au plan de Francfort. Une orientation opposée est également possible.

L'axe Z_{H} (premier axe) est parallèle à la direction de regard primaire lorsque l'individu 1 est dans une position naturelle, c'est-à-dire en posture de regard primaire. Dans le cas particulier décrit ici, l'axe Z_{H} est situé dans le plan vertical PV de la tête 4 de l'individu 1 et est parallèle au plan de Francfort. Dans d'autres cas où la tête de l'individu présente un angle de lacet, cet axe Z_{H} peut ne pas être situé dans le plan vertical. L'axe Z_{H} s'étend ici dans une direction s'éloignant de la tête 4 de l'individu 1 (vers l'arrière).

L'axe Y_{H} (troisième axe) s'étend, quant à lui, dans le plan sagittal vertical PV de la tête 4 de l'individu 1 et est perpendiculaire au plan de Francfort. L'axe Y_{H} est donc bien perpendiculaire à l'axe X_{H} et à l'axe Z_{H}. Il est ici orienté vers le haut, de sorte que le repère R_{CRO} est direct.

On notera que le repère R_{CRO} est lié à la tête 4 de l'individu 1 et que donc ce repère R_{CRO} bouge avec la tête 4 de l'individu 1, la position et l'orientation de ce repère R_{CRO} changeant par rapport à un repère absolu ou de référence (par exemple un repère lié à la pièce dans laquelle l'individu effectue le test visuel) qui ne serait pas lié à la tête 4 de l'individu 1 en fonction des mouvements de la tête 4 de l'individu 1.

On notera que la détermination des positions des centres de rotation CROD, CROG peut être réalisée selon le principe connu en soi et exposé par exemple dans le document FR 2914173, dont un équivalent en anglais est le document US 2010/0128220.

Lors de cette détermination des centres de rotation CROD, CROG, l'individu 1 porte, sur sa tête 4, solidaire de la tête 4, un système de repérage (référentiel métrologique) ou « *clip* » qui comprend des éléments de repérage (marqueurs) détectables lors d'une capture d'image de la tête 4 de l'individu 1.

En résumé, on capture au moins deux images de la tête 4 de l'individu 1 au moyen d'un appareil de capture d'images :
- une première image lorsque l'individu regarde l'appareil de capture d'images en se positionnant de face, en regardant au loin droit devant (posture de regard primaire), et
- une deuxième image lorsque l'individu regarde l'appareil de capture d'images en se positionnant de trois-quarts.

À partir d'un traitement des deux images capturées (voir document FR 2914173), on déduit les positions des centres de rotation CROD, CROG dans un référentiel lié au système de repérage.

Il est alors possible de déterminer le centre de rotation « cyclope », qui est l'isobarycentre des deux centres de rotation CROD, CROG précédemment déterminés.

Pour la détermination de la posture de regard primaire, on réutilise les positions des centres de rotation CROD, CROG ainsi que la première image capturée de face. On peut également prévoir de compenser l'inclinaison de la tablette 10 lors de cette dernière détermination.

On a représenté sur la figure 8 la direction de regard DR joignant le CRO cyclope à la cible 20 positionnée ici sur la dernière position cible du protocole de suivi visuel ainsi que le repère R_{CRO} lié à la tête 4 de l'individu 1 avec ses trois axes principaux X_{H}, Y_{H}, Z_{H}.

On a également représenté sur cette figure 8 les directions de regard référencées respectivement DRD et DRG correspondant aux directions de regard pour l'œil droit OD et l'œil gauche OG de l'individu 1.

Une fois que l'on a choisit le repère lié à la tête 4 de l'individu 1, ici le repère R_{CRO}, on peut, pour chaque position cible 30 de la cible 20 observée sur l'écran 11 de la tablette 10, déterminer les coordonnées de ces positions cibles dans ce repère R_{CRO}.

À cet effet, lors de l'étape de mesure de la méthode de détermination :
- on capture, au moyen de la caméra frontale 13 du dispositif de test 10 tournée vers la tête 4 de l'individu 1, des images d'une partie de la tête 4 de l'individu 1 observant chaque position cible 30, chaque position cible 30 pouvant être prédéterminée dans un repère lié à la caméra frontale 13,
- on mémorise ces images en association avec les coordonnées, exprimées dans ce repère lié à la caméra frontale 13, de la position cible 30 observée par l'individu 1, et
- on détermine, à partir des images capturées et des coordonnées associées de la position cible 30 observée, les coordonnées du repère R_{CRO} lié à la tête 4 de l'individu 1 dans le repère lié à l'appareil de capture d'image 13 ou les coordonnées des directions de regard DR de l'individu 1 dans le repère R_{CRO} lié à la tête 4 de l'individu 1.

Un repère lié à la caméra frontale 13 peut être par exemple le repère R_{SCR} de l'écran 11 (voir figure 5 par exemple) qui a pour origine le coin supérieur gauche 90 de l'écran 11 et pour axes les deux axes 91, 92 perpendiculaires entre eux et dirigés selon les colonnes et les lignes de l'écran 11.

Avantageusement, la caméra frontale 13 déclenche une capture d'image de la tête 4 de l'individu 1 avec un décalage de capture par rapport au moment où la cible 20 s'affiche aux positions cibles 30 prédéterminées du protocole de suivi visuel sur l'écran 11. Ce décalage peut être nul, ou bien de préférence faible, par exemple inférieur à 200 millisecondes. Ceci permet de prendre en compte le temps de réaction et de déplacement des yeux 3 de l'individu 1 lors d'un changement de position 30 de la cible 20 sur l'écran 11.

Selon une variante, la caméra frontale peut également réaliser une séquence vidéo en continu, par exemple à une cadence de vingt images par seconde, et extraire de la séquence vidéo la meilleure image donnant la meilleure information sur le comportement visuel de l'individu lors de l'affichage de la cible à la position cible correspondante.

Chaque image capturée par la caméra frontale 13 de la tablette 10 correspond ainsi à une position cible 30 prédéterminée de la cible 20 visuellement prépondérante, dont la position 30 dans le repère R_{SCR} lié à l'appareil de capture d'image 13 est parfaitement connue.

Pour déterminer les coordonnées du repère R_{CRO} lié à la tête 4 de l'individu 1 dans le repère lié à l'appareil de capture d'image 13 ou les coordonnées des directions de regard DR de l'individu 1 dans le repère R_{CRO} lié à la tête 4 de l'individu 1, il est prévu des moyens de traitement d'images de la tablette 10, constitués par exemple par le processeur de la tablette 10, qui détecte dans les images capturées de la tête 4 de l'individu 1 les marqueurs du clip porté par l'individu 1 sur sa tête 4.

On détermine alors pour chaque image capturée, c'est-à-dire pour chaque position cible 30 de la cible 20 du protocole de suivi visuel, la position et l'orientation du clip dans le repère R_{SCR} lié à la caméra frontale 13 par exemple en utilisant le procédé décrit dans le document US 2010/0128220.

Les positions des centres de rotation CROD, CROG des yeux de l'individu 1 par rapport au clip étant connues, la position (coordonnées spatiales) et l'orientation (coordonnées angulaires) du repère R_{CRO} lié à la tête 4 de l'individu 1 sont également connues par rapport au clip.

Ceci est d'ailleurs illustré sur la figure 8 où l'on a représenté le repère R_{CRO} avec son origine au centre de rotation cyclope CRO_{C} (isobarycentre des centres de rotation CROD, CROG) et ses axes X_{H}, Y_{H}, Z_{H}.

Ainsi, par un changement de repère, il est possible de déterminer, pour chaque position cible 30 de la cible 20 du protocole de suivi visuel, la position et l'orientation du repère R_{CRO} lié à la tête 4 de l'individu 1 dans le repère R_{SCR} lié à la caméra frontale 13 de la tablette 10.

On peut également déterminer, pour chaque position cible 30 de la cible 20 du protocole de suivi visuel, les directions de regard DR de l'individu 1 dans le référentiel R_{CRO} lié à la tête 4 de l'individu 1, ces directions de regard DR joignant ici le centre de rotation cyclope CRO_{C}, origine du repère R_{CRO} lié à la tête 4 de l'individu 1, à la cible 20.

On peut enfin réexprimer, à partir des positions et orientations de la tête 4 ou des directions de regard DR de l'individu 1, les positions cibles 30 de la cible 20 dans le repère R_{CRO} lié à la tête 4 de l'individu 1.

Ces positions cibles 30 dans le repère R_{CRO} lié à la tête 4 de l'individu 1 sont des données représentatives des directions de regard DR mesurées de l'individu 1 lors du protocole de suivi visuel.

On peut ainsi déterminer, après l'étape de mesure, une direction de regard de référence en fonction de ces données représentatives.

Dans certains modes de réalisation, la direction de regard de référence correspond à une direction d'observation de l'individu d'une cible éloignée (vision de loin) quand l'individu est dans une posture naturelle.

Dans le mode de réalisation décrit ici, la direction de regard de référence est une direction de regard moyenne de l'individu 1 au cours du test visuel.

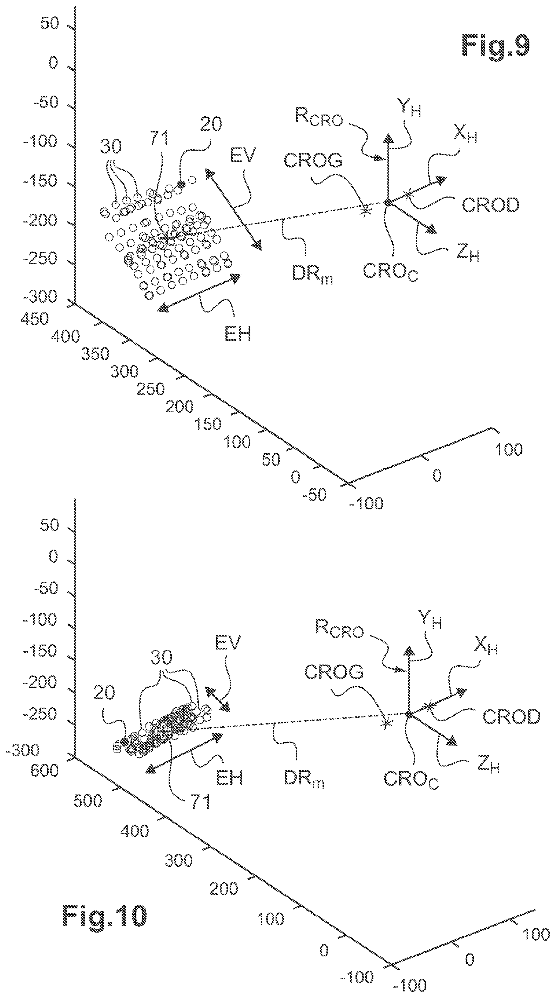

Comme représentée sur les figures 9 et 10, cette direction de regard moyenne, ci-après référencée DRₘ, est choisie de préférence comme étant la droite reliant le CRO cyclope CRO_{C} au barycentre 71 des positions cibles 30.

En variante, la direction de regard moyenne peut être définie à partir du centre de rotation droit CROD, du centre de rotation gauche CROG, du centre de rotation de l'œil directeur, ou bien encore du centre de rotation de l'œil dominant.

En variante encore, la direction de regard moyenne est choisie ici comme étant la droite reliant un centre de rotation de l'œil gauche ou droit de l'individu, ou un barycentre desdits centres de rotation, à une position cible dans le repère lié à la tête de l'individu.

Compte tenu du fait non seulement que la position et l'orientation de la tête 4 de l'individu 1 change au cours du protocole de test visuel par rapport au repère R_{SCR} lié à l'appareil de capture d'image 13 mais aussi que l'individu 1 modifie la position et l'orientation de la tablette 10 au cours du test visuel, on comprend que les positions cibles 30 de la cible 20 dans le repère R_{CRO} lié à la tête 4 de l'individu 1 renseignent sur le comportement visuel de l'individu 1, en particulier sur sa propension à bouger ses yeux 3 lors de la lecture d'un texte.

En effet, si l'individu 1 suit le protocole de suivi visuel en modifiant beaucoup sa direction de regard DR, alors les positions cibles 30 de la cible 20 dans le repère R_{CRO} lié à la tête 4 de l'individu 1 sont agencées de manière relativement semblable aux positions cibles 30 de la cible 20 dans le repère R_{SCR} lié à la caméra frontale 13. Ceci est le cas de la figure 9.

À l'inverse, si l'individu 1 suit le protocole de suivi visuel en conservant une direction de regard DR *quasi* fixe, alors les positions cibles 30 de la cible 20 dans le repère R_{CRO} lié à la tête 4 de l'individu 1 sont regroupées. Ceci est le cas de la figure 10.

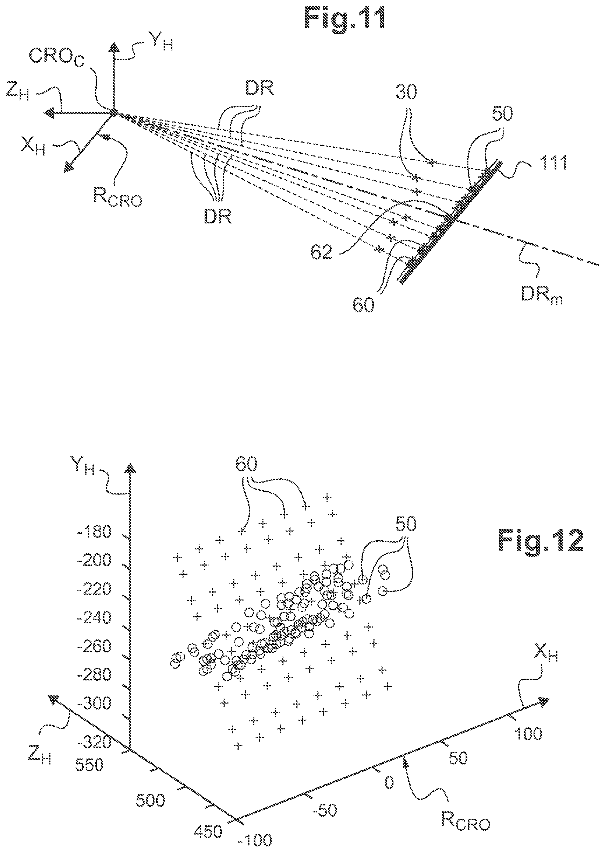

La méthode de détermination de l'invention comporte par ailleurs une étape de positionnement, par rapport à la direction de regard de référence DRₘ, de positions cibles mesurées 50 (voir figure 11) qui sont déterminées à partir des directions de regard DR de l'individu 1 mesurées au cours du test visuel lorsque l'individu 1 suit les positions cibles 30 de la cible 20 disposées sur l'écran 11 de la tablette 10.

De préférence, lors de cette étape de positionnement, on détermine également une surface d'affichage fictive 111 orientée, par rapport à la direction de regard de référence DRₘ, selon une orientation moyenne de l'écran 11 lors du test visuel.

L'orientation moyenne peut par exemple tenir compte des angles d'inclinaison et/ou de tangage moyens avec lesquels l'individu 1 tient la tablette 10 entre ses mains 2 au cours du test visuel.

Comme représenté sur la figure 11, on détermine également lors de l'étape de positionnement, les positions cibles mesurées 50 (symboles « • » sur la figure 8) comme les intersections des directions de regard DR de l'individu 1 au cours du test visuel et de la surface d'affichage fictive 111.

En d'autres termes, les positions cibles mesurées 50 correspondent aux projections des positions cibles 30, le long des directions de regard DR associées à ces positions cibles 30.

Dans un mode de réalisation préféré, la méthode de détermination comporte une étape additionnelle de positionnement.

Lors de cette étape additionnelle de positionnement, on positionne, par rapport à la direction de regard de référence, ici la direction de regard moyenne DRₘ, des positions cibles théoriques 60 (symboles « + » sur la figure 8) dont les dispositions relatives les unes par rapport aux autres sont identiques aux dispositions relatives des positions cibles 30 sur la surface d'affichage 11 (écran) de la tablette 10.

De préférence, on positionne ces positions cibles théoriques 60 de sorte que leur barycentre 62 se trouve sur la direction de regard de référence DRₘ.

Ainsi, à l'issue des étapes de positionnement décrites ci-dessus, on a déterminé, sur la surface d'affichage fictive 111, les coordonnées des positions cibles mesurées 50, et les coordonnées des positions cibles théoriques 60, dans le repère R_{CRO} lié à la tête 4 de l'individu 1. Ceci est illustré sur la figure 12 des dessins.

Des paramètres de comportement visuel de l'individu 1 lors du protocole de suivi visuel peuvent être déduits des positions cibles mesurées 50 et des positions cibles théoriques 60.

En effet, on peut déjà déterminer un premier paramètre de comportement visuel correspondant à la position (coordonnées) du barycentre (ci-après référencé NVB pour « *Near-Vision Behaviour* » en anglais) des positions cibles 30 dans le repère R_{CRO} lié à la tête 4 de l'individu 1. Ce barycentre NVB renseigne notamment sur la direction de regard moyenne DRₘ de l'individu 1 (cf. ci-dessus) lors du test visuel.

Par ailleurs, comme expliqué ci-dessus en référence aux figures 9 et 10, on comprend que la distribution (position et étalement) des positions cibles mesurées 50 par rapport aux points cibles théoriques 60, dont la distribution sur la surface d'affichage fictive 111 est fixée par celle des positions cibles 30 sur l'écran 11, renseigne sur la tendance de l'individu 1 à bouger la tête 4 et/ou les yeux 3 lors d'une tâche de lecture.

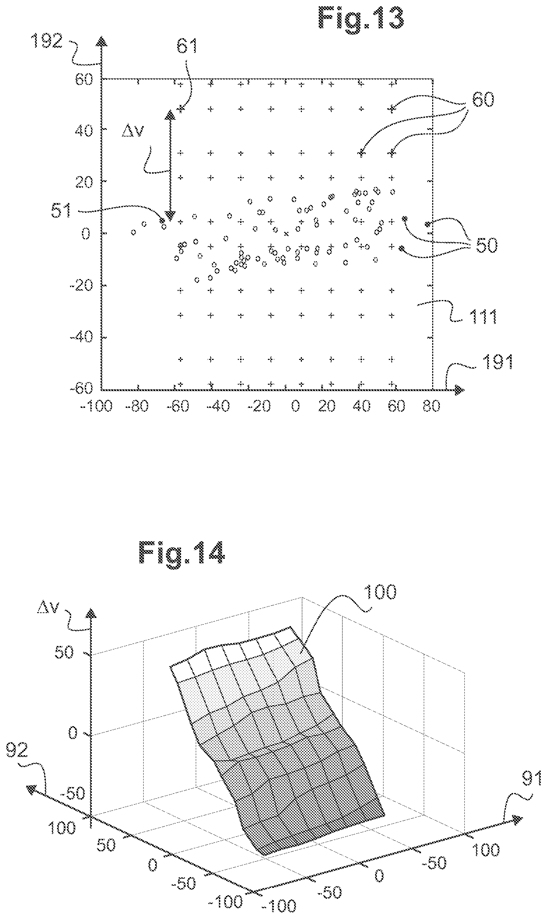

Ainsi, dans un autre mode de réalisation décrit en référence aux figures 13 à 16, l'étape de déduction de la méthode de détermination comporte de préférence une comparaison des positions cibles théoriques 60 et des positions cibles mesurées 50 dans le repère R_{CRO} lié à la tête 4 de l'individu 1. Cette comparaison permet de déduire un ou plusieurs paramètres de comportement visuel recherchés, en particulier des paramètres de comportement visuel de l'individu 1 qui sont représentatifs de l'étalement vertical EV et de l'étalement horizontal EH (voir figure 6) des positions cibles 30 dans le repère R_{CRO} lié à la tête 4 de l'individu 1. L'étalement vertical EV, respectivement l'étalement horizontal EH, est en effet représentatif de la propension de l'individu 1 à bouger ses yeux de haut en bas (ou de bas en haut), respectivement de gauche à droite (ou de droite à gauche), lors de la tâche visuelle.

Dans un mode de réalisation préféré, cette comparaison peut comprendre la détermination d'écarts entre les positions cibles théoriques 60 et les positions cibles mesurées 50 selon une direction privilégiée de la surface fictive 111. Ceci est illustré sur les figures 13 à 16.

En particulier, on a représenté sur la figure 13 la surface d'affichage fictive 111 munie d'axes 191, 192 orientés et normés de manière identiques aux axes 91, 92 de l'écran 11 (surface d'affichage réelle), les positions cibles mesurées 50 (symboles « **•** ») ainsi que les positions cibles théoriques 60 (symboles « + ») correspondantes.

On peut choisir par exemple comme direction privilégiée de la surface fictive 111, la direction verticale de l'axe 192.

Alors, pour chaque couple formé d'une position cible mesurée 51 et d'une position cible théorique 61 correspondant à la même position cible 30 du protocole de suivi visuel, on calcule un écart vertical, noté ici Δv, correspondant à la distance, selon la direction verticale, entre la position cible mesurée 51 et la position cible théorique 61 dudit couple.

On peut alors représenter (voir figure 14), pour chaque position cible 30 correspondant à un couple, l'ensemble des écarts verticaux Δv dans le repère R_{SCR} lié à la surface d'affichage 11 réelle. Cet ensemble est représenté par la surface 100 de la figure 11.

On pourrait également choisir une direction horizontale privilégiée (selon l'axe 191 de la figure 13) et calculer des écarts horizontaux plutôt que verticaux.

De manière avantageuse, on réalise un traitement statistique des écarts calculés pour déterminer le paramètre de comportement visuel.

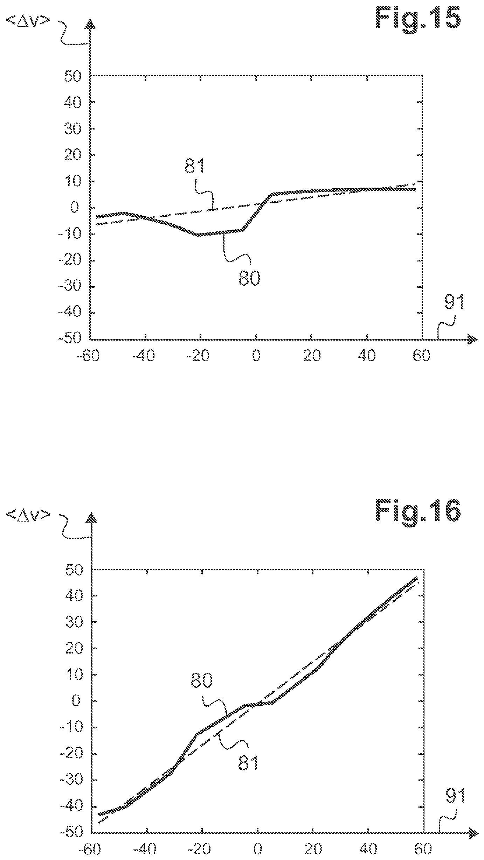

Ce traitement statistique peut par exemple comprendre les opérations suivantes :
- réaliser une moyenne < Δv > par ligne d'affichage, des écarts verticaux Δv. On obtient alors des courbes mesurées 80 telles que représentées sur les figures 15 et 16 où la moyenne < Δv > est fonction de l'index de colonne ;
- effectuer une régression linéaire afin de trouver une droite approchante 81 qui minimise l'écart avec les courbes mesurées 80.

Le coefficient directeur de cette droite approchante 81 fournit un paramètre de comportement visuel de l'individu 1 lors du protocole de test visuel.

Ce coefficient directeur est en particulier déterminé pour se situer entre 0 et 1. Pour cela, on détermine une valeur seuil minimale et une valeur seuil maximale permettant, pour des facilités d'utilisation, de normer le coefficient. Ainsi on recalcule le rapport (coefficient directeur - valeur minimale / valeur maximale - valeur minimale).

Les valeurs maximale et minimale peuvent être obtenues à partir d'une distribution de coefficients directeurs préenregistrés ou obtenues à partir de plusieurs individus.

En effet, lorsque ce coefficient directeur est faible (cas de la figure 12 avec un coefficient de 0,17), cela signifie que la moyenne des écarts entre les positions cibles mesurées 50 et les positions cibles théoriques 60 est faible. Ceci correspond au comportement visuel d'un individu 1 bougeant beaucoup les yeux 3 lors du test visuel.

À l'inverse, lorsque ce coefficient directeur est élevé (cas de la figure 13 avec un coefficient de 0,83), cela signifie que la moyenne des écarts entre les positions cibles mesurées 50 et les positions cibles théoriques 60 est élevée. Ceci correspond au comportement visuel d'un individu 1 bougeant peu les yeux 3 lors du test visuel.

## Revendications

1. Procédé de mesure de la réfraction d'un individu au moyen d'un appareil de mesure de la réfraction, comportant :
a) une étape initiale de détermination, sans équipement initial de correction visuelle, d'au moins une valeur initiale d'un paramètre visuo-postural dudit individu, relatif au positionnement de sa tête et/ou de son ou ses yeux en situation ou activité de vision ;
b) une étape de traitement de ladite valeur initiale du paramètre visuo-postural déterminée à l'étape a) pour déduire au moins une valeur initiale d'un paramètre de réglage dudit appareil de mesure de la réfraction, ledit paramètre de réglage étant associé audit paramètre visuo-postural ;
c) une étape de réglage dudit appareil de mesure de la réfraction selon ladite valeur initiale déduite à l'étape b) ; et
d) une étape de mesure de la réfraction de l'individu au moyen dudit appareil de mesure de la réfraction réglé à l'étape c),
e) une étape d'équipement de l'individu avec un équipement de test de correction visuelle adapté à corriger la réfraction mesurée à l'étape d) ;
f) une étape supplémentaire de détermination, avec ledit équipement de test, d'au moins une valeur supplémentaire dudit paramètre visuo-postural dudit individu ;
g) une étape supplémentaire de traitement de ladite valeur supplémentaire du paramètre visuo-postural déterminée à l'étape f) pour déduire au moins une valeur suivante dudit paramètre de réglage associé audit paramètre visuo-postural ;
h) une étape de comparaison desdites valeurs initiale et suivante dudit paramètre de réglage, et selon lequel :
- lorsque la comparaison de l'étape h) indique que lesdites valeurs initiale et suivante dudit paramètre de réglage diffèrent au-delà d'un seuil de différence prédéterminé, on répète les étapes c) et d) du procédé avec ladite valeur suivante du paramètre de réglage pour mesurer une nouvelle valeur de la réfraction de l'individu au moyen dudit appareil de mesure de la réfraction réglé selon cette valeur suivante du paramètre de réglage et on enregistre ladite nouvelle valeur de la réfraction et la valeur de la réfraction mesurée à l'étape d) ; et
- lorsque la comparaison de l'étape h) indique que lesdites valeurs initiale et supplémentaire diffèrent en deçà dudit seuil de différence prédéterminé, on enregistre ladite valeur supplémentaire déterminée à l'étape f) et ladite réfraction mesurée à l'étape d).

2. Procédé de mesure selon la revendication 1 , selon lequel ledit paramètre visuo-postural de l'individu déterminé à l'étape a) comporte l'un des paramètres suivants :
- une posture naturelle de la tête de l'individu ;
- un paramètre de comportement visuel en posture naturelle ;
- un coefficient œil / tête ;
- une distance de lecture en vision de près ;
- une valeur de décalage d'un point de fixation par rapport au plan médian de la tête de l'individu ;
- un angle d'abaissement du regard ;
- un paramètre de convergence des deux yeux en vision de près ;
- une direction de regard.

3. Procédé de mesure selon l'une des revendications 1 à 2, selon lequel :
- à l'étape a), on équipe ledit individu d'une monture de lunettes, non pourvue de lentilles ophtalmiques correctives ; et
- à l'étape c), on règle également ledit appareil de mesure selon au moins l'un des paramètres complémentaires de réglages suivants :
- une distance verre-œil ;
- un angle pantoscopique de ladite monture ;
- un paramètre de galbe de ladite monture ;
- un paramètre de centrage de lentilles ophtalmiques dans ladite monture de lunettes.

4. Procédé de mesure selon l'une des revendications 1 à 3, selon lequel, ledit paramètre visuo-postural de l'individu étant identique audit paramètre de réglage de l'appareil de mesure de la réfraction réglé à l'étape c), ledit traitement de l'étape b) consiste à égaliser ladite valeur initiale du paramètre de réglage à ladite valeur initiale du paramètre visuo-postural.

5. Procédé de conception optique d'une lentille ophtalmique destiné à un individu comportant les étapes suivantes :
i) déterminer une valeur de la réfraction de l'individu grâce à la mise en œuvre du procédé de mesure selon l'une des revendications 1 à 4 ; et
ii) déterminer le profil optique de ladite lentille ophtalmique en fonction de ladite valeur de réfraction mesurée.

6. Système pour la mise en œuvre du procédé de mesure de la réfraction d'un individu selon l'une des revendications 1 à 4, ledit système comprenant :
- un dispositif de test visuel adapté à évaluer ledit paramètre visuo-postural de l'individu ;
- des moyens de calcul adaptés pour déduire une valeur d'un paramètre de réglage à partir d'une valeur du paramètre visuo-postural de l'individu évalué par le dispositif de test visuel ; et
- un appareil de mesure de la réfraction adapté à être réglé selon ladite valeur du paramètre de réglage déduite par les moyens de calculs et à mesurer la réfraction de l'individu.

7. Programme d'ordinateur comprenant des instructions de code pour la mise en œuvre de l'étape b) du procédé de mesure de la réfraction d'un individu selon l'une des revendications 1 à 4 lorsque ledit programme est exécuté sur les moyens de calcul du système de la revendication 6.

## Patentansprüche

1. Verfahren zur Messung der Refraktion einer Person mit Hilfe eines Apparats zur Messung der Refraktion, umfassend:
a) einen anfänglichen Schritt des Bestimmens, ohne eine anfängliche Ausstattung zur visuellen Korrektur, mindestens eines Anfangswerts eines visuell-posturalen Parameters der Person bezüglich der Positionierung ihres Kopfes und/oder ihres Auges/ihrer Augen in einer sehenden Situation oder bei einer sehenden Tätigkeit;
b) einen Schritt des Verarbeitens des in Schritt a) bestimmten Anfangswerts des visuell-posturalen Parameters, um mindestens einen Anfangswert eines Einstellparameters für den Apparat zur Messung der Refraktion abzuleiten, wobei der Einstellparameter mit dem visuell-posturalen Parameter assoziiert ist;
c) einen Schritt des Einstellens des Apparats zur Messung der Refraktion gemäß dem in Schritt b) abgeleiteten Anfangswert; und
d) einen Schritt des Messens der Refraktion der Person mit Hilfe des in Schritt c) eingestellten Apparats zur Messung der Refraktion,
e) einen Schritt des Ausstattens der Person mit einer Testausstattung zur visuellen Korrektur, die dazu angepasst ist, die in Schritt d) gemessene Refraktion zu korrigieren;
f) einen zusätzlichen Schritt des Bestimmens, mit der Testausstattung, mindestens eines zusätzlichen Werts des visuell-posturalen Parameters der Person;
g) einen zusätzlichen Schritt des Verarbeitens des in Schritt f) bestimmten zusätzlichen Werts des visuell-posturalen Parameters, um mindestens einen Folgewert des Einstellparameters, der mit dem visuell-posturalen Parameter assoziiert ist, abzuleiten;
h) einen Schritt des Vergleichens des Anfangs- und des Folgewerts des Einstellparameters, und wobei:
- wenn der Vergleich des Schritts h) angibt, dass sich der Anfangs- und der Folgewert des Einstellparameters oberhalb einer vorbestimmten Differenzschwelle unterscheiden, Wiederholen der Schritte c) und d) des Verfahrens mit dem Folgewert des Einstellparameters, um einen neuen Wert der Refraktion der Person mit Hilfe des gemäß diesem Folgewert des Einstellparameters eingestellten Apparats zur Messung der Refraktion zu messen, und Speichern des neuen Werts der Refraktion und des in Schritt d) gemessenen Werts der Refraktion; und
- wenn der Vergleich des Schritts h) angibt, dass sich der Anfangs- und der zusätzliche Wert unterhalb der vorbestimmten Differenzschwelle unterscheiden, Speichern des in Schritt f) bestimmten zusätzlichen Werts und der in Schritt d) gemessenen Refraktion.

2. Messverfahren nach Anspruch 1, wobei der in Schritt a) bestimmte visuell-posturale Parameter der Person einen der folgenden Parameter umfasst:
- eine natürliche Haltung des Kopfes der Person;
- einen Sehverhaltensparameter in der natürlichen Haltung;
- einen Auge/Kopf-Koeffizienten;
- einen Leseabstand beim Nahsehen;
- einen Versatzwert eines Fixierungspunktes in Bezug auf die Medianebene des Kopfes der Person;
- einen Winkel des Absenkens des Blicks;
- einen Konvergenzparameter der zwei Augen beim Nahsehen;
- eine Blickrichtung.

3. Messverfahren nach einem der Ansprüche 1 bis 2, wobei:
- in Schritt a) die Person mit einem Brillengestell ausgestattet wird, das nicht über korrektive Brillengläser verfügt; und
- in Schritt c) der Messapparat ferner gemäß mindestens einem der folgenden komplementären Einstellparameter eingestellt wird:
- einem Glas-Auge-Abstand;
- einem pantoskopischen Winkel des Gestells;
- einem Wölbungsparameter des Gestells;
- einem Parameter zum Zentrieren von Brillengläsern in dem Brillengestell.

4. Messverfahren nach einem der Ansprüche 1 bis 3, wobei, da der visuell-posturale Parameter der Person zu dem Einstellparameter des in Schritt c) eingestellten Apparats zur Messung der Refraktion identisch ist, das Verarbeiten des Schritts b) darin besteht, den Anfangswert des Einstellparameters mit dem Anfangswert des visuell-posturalen Parameters abzugleichen.

5. Verfahren für das optische Design eines Brillenglases, das für eine Person bestimmt ist, umfassend die folgenden Schritte:
i) Bestimmen eines Werts der Refraktion der Person mit Hilfe der Umsetzung des Messverfahrens nach einem der Ansprüche 1 bis 4; und
ii) Bestimmen des optischen Profils des Brillenglases in Abhängigkeit von dem gemessenen Refraktionswert.

6. System zur Umsetzung des Verfahrens zur Messung der Refraktion einer Person nach einem der Ansprüche 1 bis 4, wobei das System Folgendes beinhaltet:
- eine visuelle Testvorrichtung, die dazu angepasst ist, den visuell-posturalen Parameter der Person zu bestimmen;
- Rechenmittel, die dazu angepasst sind, einen Wert eines Einstellparameters anhand eines Werts des mit der visuellen Testvorrichtung bestimmten visuell-posturalen Parameters der Person abzuleiten; und
- einen Apparat zur Messung der Refraktion, der dazu angepasst ist, gemäß dem durch die Rechenmittel abgeleiteten Wert des Einstellparameters eingestellt zu werden und die Refraktion der Person zu messen.

7. Computerprogramm, das Codeanweisungen beinhaltet, die bei Ausführung des Programms auf den Rechenmitteln des Systems nach Anspruch 6 den Schritt b) des Verfahrens zur Messung der Refraktion einer Person nach einem der Ansprüche 1 bis 4 umsetzen.

## Claims

1. Method for measuring the refraction of an individual by means of a refraction-measuring apparatus, comprising:
a) an initial step of determining, without initial vision-correcting equipment, at least one initial value of a visuo-postural parameter of said individual, relating to the positioning of their head and/or of their eye or eyes in a vision situation or activity;
b) a step of processing said initial value of the visuo-postural parameter determined in step a) in order to deduce at least one initial value of an adjustment parameter of said refraction-measuring apparatus, said adjustment parameter being associated with said visuo-postural parameter;
c) a step of adjusting said refraction-measuring apparatus depending on said initial value deduced in step b); and
d) a step of measuring the refraction of the individual by means of said refraction-measuring apparatus adjusted in step c);
e) a step of equipping the individual with an item of vision-correcting test equipment suitable for correcting the refraction measured in step d);
f) an additional step of determining, with said test equipment, at least one additional value of said visuo-postural parameter of said individual;
g) an additional step of processing said additional value of the visuo-postural parameter determined in step f) in order to deduce at least one following value of said adjustment parameter associated with said visuo-postural parameter;
h) a step of comparing said initial and following values of said adjustment parameter, and wherein:
- when the comparison of step h) indicates that said initial and following values of said adjustment parameter differ by more than a predetermined difference threshold, steps c) and d) of the method are repeated with said following value of the adjustment parameter in order to measure a new value of the refraction of the individual by means of said refraction-measuring apparatus adjusted depending on this following value of the adjustment parameter and said new value of the refraction and the value of the refraction measured in step d) are recorded; and
- when the comparison of step h) indicates that said initial and additional values differ by less than said predetermined difference threshold, said additional value determined in step f) and said refraction measured in step d) are recorded.

2. Measuring method according to Claim 1, wherein said visuo-postural parameter of the individual determined in step a) comprises one of the following parameters:
- a natural posture of the head of the individual;
- a visual behaviour parameter in natural posture;
- an eye/head coefficient;
- a reading distance in near vision;
- an offset value of a point of fixation with respect to the median plane of the head of the individual;
- an angle of lowering of the gaze;
- a parameter of convergence of the two eyes in near vision;
- a gaze direction.

3. Measuring method according to either of Claims 1 and 2, wherein:
- in step a), said individual is equipped with a spectacle frame, not provided with corrective ophthalmic lenses; and
- in step c), said measuring apparatus is also adjusted depending on at least one of the following complementary adjustment parameters:
- a lens-eye distance;
- a pantoscopic angle of said frame;
- a wrap parameter of said frame;
- a parameter of centring of ophthalmic lenses in said spectacle frame.

4. Measuring method according to one of Claims 1 to 3, wherein, said visuo-postural parameter of the individual being identical to said adjustment parameter of the refraction-measuring apparatus adjusted in step c), said processing of step b) consists in making said initial value of the adjustment parameter equal to said initial value of the visuo-postural parameter.

5. Optical design method for designing an ophthalmic lens intended for an individual, comprising the following steps:
i) determining a value of the refraction of the individual by virtue of the implementation of the measuring method according to one of Claims 1 to 4; and
ii) determining the optical profile of said ophthalmic lens depending on said measured refraction value.

6. System for implementing the method for measuring the refraction of an individual according to one of Claims 1 to 4, said system comprising:
- a vision-testing device suitable for evaluating said visuo-postural parameter of the individual;
- computing means suitable for deducing a value of an adjustment parameter from a value of the visuo-postural parameter of the individual evaluated by the vision-testing device; and
- a refraction-measuring apparatus suitable for being adjusted depending on said adjustment-parameter value deduced by the computing means and for measuring the refraction of the individual.

7. Computer program comprising code instructions for implementing step b) of the method for measuring the refraction of an individual according to one of Claims 1 to 4 when said program is executed on the computing means of the system of Claim 6.
